# EUROPEAN PATENT SPECIFICATION

(11) **EP 4 227 680 B1**
(45) Date of publication and mention of the grant of the patent: **29.04.2026**
(21) Application number: 22382103.4
(22) Date of filing: 09.02.2022
(51) Int. Cl.: G01N 33/483, G01N 3/00, C12M 1/42

(54) **METHOD FOR GENERATING AND CONTROLLING COMPLEX STRAIN PATTERNS ON BIOLOGICAL MATERIALS, MAGNETO-MECHANICAL STIMULATION SYSTEM FOR GENERATING COMPLEX STRAIN PATTERNS IN BIOLOGICAL MATERIALS**
VERFAHREN ZUR ERZEUGUNG UND STEUERUNG KOMPLEXER DEHNUNGSMUSTER AUF BIOLOGISCHEN MATERIALIEN, MAGNETOMECHANISCHES STIMULATIONSSYSTEM ZUR ERZEUGUNG KOMPLEXER DEHNUNGSMUSTER IN BIOLOGISCHEN MATERIALIEN
PROCÉDÉ DE PRODUCTION ET DE CONTRÔLE DE MOTIFS DE DÉFORMATION COMPLEXES SUR DES MATÉRIAUX BIOLOGIQUES, SYSTÈME DE STIMULATION MAGNÉTO-MÉCANIQUE POUR LA GÉNÉRATION DE MOTIFS DE DÉFORMATION COMPLEXES DANS DES MATÉRIAUX BIOLOGIQUES

(43) Date of publication of application: 16.08.2023
(73) Proprietor: Universidad Carlos III de Madrid, 28919 Leganes (Madrid) (ES)
(72) Inventor: García González, Daniel, 28919 Leganes (Madrid) (ES); Moreno Mateos, Miguel Ángel, 28919 Leganes (Madrid) (ES); Muñoz Barrutia, María Arrate, 28919 Leganes (Madrid) (ES); Nuñez Sardinha, Emanuel David, 28919 Leganes (Madrid) (ES)
(74) Representative: Pons IP

(56) References cited:
- US-A1- 2005 107 870
- US-A1- 2015 093 823
- US-A1- 2021 040 427
- MAYER MATTHIAS ET AL: "Ultra-Soft PDMS-Based Magnetoactive Elastomers as Dynamic Cell Culture Substrata", vol. 8, no. 10, 18 October 2013 (2013-10-18), pages e76196, XP055936724, Retrieved from the Internet <URL:https://journals.plos.org/plosone/article/file?id=10.1371/journal.pone.0076196&type=printable> [retrieved on 20220629], DOI: 10.1371/journal.pone.0076196
- MORENO M A ET AL: "New experimental insights into magneto-mechanical rate dependences of magnetorheological elastomers", COMPOSITES PART B, ELSEVIER, AMSTERDAM, NL, vol. 224, 18 July 2021 (2021-07-18), XP086790434, ISSN: 1359-8368, [retrieved on 20210718], DOI: 10.1016/J.COMPOSITESB.2021.109148
- ELHAJJAR RANI ET AL: "Magnetostrictive polymer composites: Recent advances in materials, structures and properties", vol. 97, 24 February 2018 (2018-02-24), GB, pages 204 - 229, XP055810663, ISSN: 0079-6425, Retrieved from the Internet <URL:https://pegoretti.dii.unitn.it/resources/Untitled-Folder/Papers/195-Elhajjar-Magnetostrictive-polymer-composites_PMS-2018.pdf> [retrieved on 20220629], DOI: 10.1016/j.pmatsci.2018.02.005

## Description

### OBJECT OF THE INVENTION

The invention refers to the field of analyzing biological materials (i.e., cellular systems, biological tissue), more particularly, analyzing biological materials subjected to mechanobiological processes that occur during complex and dynamic deformation states.

Thus, it is an object of the invention a method for generating and controlling complex strain patterns on biological materials, which allows to simulate complex and dynamic deformation states in said biological material.

Another object of the invention is a magneto-mechanical stimulation system for generating the complex strain patterns in the biological materials.

### BACKGROUND ART

Mechanobiology studies how mechanical forces modulate biological cells and tissues behavior. Biological cells and tissues are continuously subjected to mechanical stress and strain cues from their surrounding substrate. These processes can be of extreme complexity and their evaluation in a controlled environment is a current challenge.

Tremendous research efforts are dedicated to unravelling the effects of mechanics in biology such as alterations in functional responses, morphological changes and the influence on migration, growth or healing processes.

However, there are still significant limitations to evaluating biomechanical effects and applying defined mechanical stimuli for quantitative analysis.

Critical limitations are related to the isolation, combination and non-invasive control of the aimed mechanical actions affecting the biological structure of interest.

Current approaches are based on polymeric substrates for cell culture and the direct application of mechanical loading on them. However, this kind of system is limited to rather simple deformation modes and rarely allows for a combination of them (neither sequentially nor simultaneously). This limitation hinders the analysis and deep understanding of mechanical scenarios where complex deformation states evolve over time.

Examples of complex deformation states evolving along time are closed-head impacts, where heterogeneous strain distributions rapidly evolve resulting in significant alterations in physiological behavior. Also, scarring processes (e.g., cardiac, glial, or dermal) inducing stiffness gradients within the tissue also lead to heterogeneous strain patterns resulting in temporal-varying interaction forces between the extracellular matrix (ECM) and the cellular systems. Moreover, other relevant biological processes such as neural crest cell migration during development are also highly influenced by tissue stiffening and complex deformation modes associated with a gradual organization and enrichment of collagen fibers.

Another limitation of most of the current mechanical-stimulation systems is that they require direct (invasive) contact with the cellular substrate, hence risking to contaminate and introduce local damage to the matter.

More sophisticated solutions (non-direct contact with cellular substrate) include photo-activated changes in material properties but are limited by the low penetration depth of visible light in the material and surrounding medium.

To overcome these issues, some authors have proposed the use of magneto-responsive (or magneto-active) substrates to support the cells. However, these approaches were limited to overall stiffness changes and the control of different deformation modes was not allowed.

There is not in the art available methodology to conduct non-invasive mechanobiological studies under controlled complex and time-varying deformation scenarios.

Intricate physiological strain patterns, such as myocardial tissue contractions, skin Langer lines and deformations on the brain due to closed head impact, require novel experimental frameworks. There is a need not only to reproduce these patterns, but also to control and adapt them over time.

Examples of relevant prior art are US 2015/093823 A1 and Mayer M. et al: "Ultra-Soft PDMS-Based Magnetoactive Elastomers as Dynamic Cell Culture Substrata", PLOS ONE, vol. 8, no. 10, 18 October 2013, page e76196, DOI: 10.1371/journal.pone.0076196.

### DESCRIPTION OF THE INVENTION

The invention is defined by claims 1 and 5. Further embodiments are described in the dependent claims.

The invention relates to a magneto-mechanical stimulation system for non-invasive and real-time generation and control of mechanical stiffness and/or complex strain patterns within biological substrates. The system allows for dynamically imposing these mechanical variables controlling their temporal evolution and applied frequency. The system of the invention has the versatility to, fed by in vivo experimental strain distributions, reproduce in vitro a real mechanical event along time.

The stimulation system comprises a magneto-responsive substrate, which responds to external magnetic stimuli and transmits the resulting forces to a biological material cultured on it. The substrate comprises a polymeric matrix with embedded magnetic particles, preferably made of extremely soft polymers for mimicking the stiffness of biological materials (from 10¹ to 10⁵ Pa, preferably in the order of 10³ Pa). The magneto-responsive substrate is configured to hold biological material, thus, enabling the untethered control of the biological material, allowing to provide reversible mechanical changes and to control heterogeneous strain patterns in the biological material.

The non-invasive stimulation is possible thanks to the ability of the magneto-responsive substrates to respond mechanically to external magnetic fields. Preferably, the magneto-responsive substrate is a magnetorheological elastomer (MRE).

In particular, the magneto-responsive substrate can comprise a soft elastomeric matrix (e.g., such as Polydimethylsiloxane (PDMS)), filled with micron-size magnetic particles (e.g., such as carbonyl iron powder with a mean diameter of 5 µm). When imposing an external magnetic field, the particles magnetize leading to internal forces in the form of dipole-dipole interactions. These forces are transmitted to the polymeric matrix leading to mechanical deformation and/or changes in the rheological properties of the MRE material, for example varying its stiffness.

The surface in contact with the cultured cellular system could be covered with a collagen coating to enhance biocompatibility.

The stimulation system also comprises a magnetic stimulation device comprising at least one motor and at least two magnets placed around the magneto-responsive substrate, such that the at least one motor is connected to the magnets to displace them. The magnetic stimulation device is configured to generate a complex strain pattern on the biological material by generating a magnetic field which acts over the magneto-responsive substrate.

The magnetic stimulation device can preferably comprise four independently controllable sets of permanent magnets surrounding the substrate, with two sets aligned along an axis. Thus, by moving the magnets closer or farther from each other, the resulting field over the region of interest is varied. The two axes lie orthogonal to control the field in each direction, with the final field obtained by superposition.

The magnetic stimulation device can comprise a rotation mechanism configured to rotate in azimuthal direction the central region holding the substrate and cellular system, thus, adding another degree of freedom. This adds to extra possibilities to the stimulation: 1) application of the possible complex deformation modes but changing the initial orientation of the substrate-cellular system; and 2) application of angular displacement while keeping a strong magnetic field with permanent magnets close to the sample, so that material twisting is induced.

The magneto-stimulation device is fitted in a holder, and preferably, in an incubator allowing for the application of magnetic fields on the magneto-responsive substrate along time.

By using a regular incubator, the biological material can be evaluated long-term at different time points by using a microscope as imaging module.

Alternatively, the system can be used directly on a microscope as imaging module to evaluate in real-time the influence of the mechanical effects on the biological material.

The resultant mechanical deformation of the MRE substrate will be determined by the final magnetic field and macroscopic magnetic interactions due to the presence of the magnets, with the magnitude determined by their relative position to the magneto-responsive substrate. Overall, local mechanical deformation of the cellular substrate can be controlled on the fly in terms of magnitude and orientation, allowing to transmit reversible forces to cells.

Therefore, the overall response of the substrate depends on different factors: the nature of the polymeric matrix and the magnetic particles, the distribution of the magnetic particles within the polymeric matrix, the mechanical boundary conditions (BCs) imposed and the direction and magnitude of external magnetic fields generated by the magnetic stimulation device.

Relating to the mechanical boundary conditions, different situations can be found:
i) If fixed boundary conditions are imposed, the substrate does not deform but experiences internal stress. Thus, if the polymeric matrix presents a nonlinear mechanical behavior, the result of the magnetic field application is a variation in apparent material stiffness of the substrate.
ii) If free boundary conditions are imposed, the internal stresses within the substrate due to the application of the magnetic field result in mechanical deformation.

Therefore, the stimulation system, comprising the magneto-responsive substrate and the magnetic stimulation device, allows to control the mechanical deformation of biological material placed on or within the substrate in a non-invasive manner, allowing at the same time for temporal programming of complex deformation modes.

With the system as described, large deformation states can be imposed on cellular substrates of around 0.5 to 3 kPa stiffness (in terms of shear modulus).

Although this stiffness range is ideal for several biological studies, softer or stiffer substrates may be needed in other scenarios. In this regard, the system allows for using magneto-active hydrogels instead of MRE if softer substrates are required. Indeed, soft enough hydrogels would allow for manufacturing magneto-active substrates enabling the use of the stimulator system in 3D models.

For the use of stiffer substrates, however, the main limitation would be the need for higher magnetic fields to reach significant mechanical deformation. This problem may be solved by using larger magnets or using specific designs for the specific experiment by optimizing (i.e., reducing) the size of the sample, resulting in much higher magnetic fields for the same magnets. Another alternative to reach higher magnetic fields is the use of electromagnets instead of permanent magnets.

Contrary to the stimulation used in the specific applications presented in this work, one may study the effect of dynamic substrate stiffness instead of mechanical deformation on cellular processes. The stimulation system developed would also allow this by imposing fixed boundary conditions. In this case, instead of changing its shape, the sample would alternatively undergo internal stress states. Therefore, using a polymeric matrix with a strong nonlinear response (i.e., substantial strain hardening), the application of the external magnetic field will result in a variation of the apparent material stiffness.

The stimulation system also comprises a computing module to guide the controlling parameters to impose mechanical deformation patterns on the substrate. Consequently, mechanical forces are transmitted to the biological material affecting its behavior.

While the magnetic fields can be straightforwardly tuned by the location of the permanent magnets, controlling the resulting mechanical deformation of the magneto-active substrate is a challenging endeavor. Such a magneto-mechanical response is determined by numerous mechanisms that develop in parallel at both micro- and macroscales. To overcome these difficulties and provide an efficient controlling system for the substrate deformation, an interface module, connected to the computing module and the imaging module, imposes the magneto-mechanical dynamic conditions on the magneto-responsive substrate by acting over the computing module.

In this regard, the computing module allows for two alternatives: 1) a method based on high fidelity simulations, i.e., finite element simulations; 2) a method based on machine learning algorithms.

The computing module can perform numerical simulations that compute, in the presence of a given magneto-responsive substrate, the effective magnetic field magnitude and direction depending on the position of the permanent magnets. Then, the subsequent deformation state within the substrate is estimated by multi-physical computational modelling taking as input the material substrate characteristics.

The model used by the system of the invention takes as inputs material parameters such as polymeric matrix stiffness, magnetic properties of the particles and particles' content, that can be controlled during the manufacturing process.

The computational model provides physical insights into the magneto-mechanical coupling and guidance during experimental designs. It defines the relative positions between permanent magnets and the MRE substrate leading to a specific mechanical deformation state.

In said model, the substrate material composition and geometry are introduced, as well as the position of the permanent magnets, to provide:
i) the spatial distribution of the magnetic fields; and
ii) the resultant local deformations within the substrate.

The finite element model explicitly accounts for the permanent magnets, MRE sample and surrounding air, and it couples nonlinear magnetics with nonlinear mechanics.

Analysis accounting for fast cellular responses requires the consideration of viscous and relaxation mechanisms.

The computing module can alternatively consist of a machine learning framework fed by a comprehensive experimental characterization of several magneto-active samples. These experiments provide for different magnetic conditions the mechanical deformation response of the magneto-active substrate. All these data are used within an optimization framework to guide the design of experiments on a given substrate. To this end, the framework takes as input the manufacturing characteristics of the substrate and a simple test under certain magnetic conditions. Then, the system identifies, from the database, the case that better fits with the response of the given substrate. From this, the framework accounts for the experimental variability expected and provides, from this reference, the magnetic conditions needed to reach in the given sample a determined strain field. This approach is highly reliable and robust compared to others based on predictive simulations, as this takes experimental data directly.

The computing module described is essential for reproducing complex strain patterns from in vivo data.

The invention is referred to a method for generating complex strain patterns or biological materials that allows for non-invasive and real-time control of complex deformation modes within a magneto-responsive substrate.

The method of the invention allows for instantaneous evaluation of mechanical effects and for influencing different biological responses while recording the whole temporal event.

For generating and control a complex strain pattern on biological materials, different magneto-responsive substrates varying their stiffness and the intensity of their magneto-mechanical coupling are extensively characterized at both macroscopic and microscopic scales, unveiling the mechanisms that govern their behavior. Then, the biological material is cultured on the substrate.

Then, a computing module act as guidance for the experimental stimulation setup by determining the position of the magnets for controlling the magnetic field generated. This provides the controlling parameters to induce desired magnetic fields on the MRE-cellular substrate, and the subsequent mechanical deformation to be transmitted to the biological material. The determination of the position of the magnets allows to obtain a defined strain pattern on the biological material. Therefore, the magnets are placed in the position determined by means of the at least one motor of the magnetic stimulation device. Then, the magnetic stimulation device is activated to generate a complex strain pattern in the magneto-responsive substrate and consequently in the biological material.

Differently from previous approaches in the literature, the system and method of the invention allow at the same time for: non-invasive (through magnetic fields) mechanical stimulation, real-time control of mechanical stimulation, and alternating (complex) deformation modes controlling local changes in both magnitude and principal strain components.

The proposed framework would lead to novel studies to understand mechanobiological mechanisms behind relevant processes that develop under complex and dynamic deformation states (e.g., traumatic brain injury, pathological skin scarring, fibrotic heart remodeling during myocardial infarction). In addition, it opens doors for novel stimulation capabilities of biological matter.

Due to the nonlinear coupling between magnetic fields and mechanical deformation, the interface module of the stimulation system can comprise a 3D finite element module to make use of a 3D finite element model for controlling and predicting the deformation patterns transmitted to the cells during the application of the magnetic field.

The system and method of the invention open the possibility of novel in vitro fundamental studies to evaluate the effects of mechanics on mechanistic-mediated biological processes. The system allows for remote modulation of complex strain conditions on the cellular substrate by applying (non-invasive) external magnetic fields.

These strain patterns can be controlled in real-time, enabling dynamic modulation of magneto-mechanical loading. In addition, the imaging setup built in this work permits visualizing the cellular process along time on the fly.

This will allow to address relevant problems within the bioengineering field such as the study of scarring processes, wound healing, cell polarization of immune cells, proliferation and migration processes in cancer progression, axonal damage due to mechanical impact or neural crest cell migration, among others.

The system described focuses on the creation of customized mechano-stimulation systems for biological matter, suitable thanks to the low magnetic permeability of biological tissues. The results obtained by the system will offer direct benefits for health purposes by paving the path to models that reproduce mechanistic-mediated biological processes, unrevealing the underlying mechanisms that take place during physiological scenarios such as traumatic brain injury, pathological skin scarring or fibrotic heart remodeling during myocardial infarction.

The system can reproduce complex mechanical processes by simulating a set of local strain patterns occurring in physiologically relevant scenarios. In this way, the proposed system allows for altering different deformation states to simulate in vitro complex biomechanical processes. Therefore, the system opens the way to understanding the mechanobiological processes that occur during complex and dynamic deformation states, e.g., in traumatic brain injury, pathological skin scarring or fibrotic heart remodeling during myocardial infarction.

### DESCRIPTION OF THE DRAWINGS

Fig. 1. Shows an exemplary embodiment of the stimulation system of the invention within an incubator placed in a microscope.
Fig. 2. Shows a method for generating and controlling complex strain patterns on biological materials.
Fig. 3. Shows the characterization of a potential substrate under uniaxial compression loading, using a magneto-mechanical rheometer, by representing the storage Young's modulus depending on the mixing ratio used for the substrate and on the magnetic field applied.
Fig. 4. Shows the characterization of the substrate under shear loading, using a magneto-mechanical rheometer, representing the storage shear modulus depending on the mixing ratio used for the substrate and on the magnetic field applied.
Fig. 5. Shows the results of the quasi-static nanoindentation test, by representing the Young's modulus depending on the mixing ratio used for the substrate.
Fig. 6. Shows the relaxation response depending on the mixing ratio used for the substrate, representing the load versus time relaxation curves conducted by nanoindentation.
Fig. 7. Shows a comparison of the multifunctional response to the complex strain patterns between experimental data and computational predictions by means of displacement fields, for an approach of 2 magnets and for two different magneto-active substrates.
Fig. 8. Shows a comparison of the multifunctional response to the complex strain patterns between experimental data and computational predictions by means of displacement fields, for an approach of 4 magnets and for two different magneto-active substrates.
Fig. 9. Shows experimental results obtained from digital image correlation of local mechanical deformation within different magneto-active substrates and for an approach of 2 magnets.
Fig. 10. Shows experimental results obtained from digital image correlation of local mechanical deformation within different magneto-active substrates and for an approach of 4 magnets.
Fig. 11. Shows a proof of concept to evaluate the versatility of the system to simulate complex heterogeneous strain distributions by showing: row A) an in vivo strain distribution within an axial view of a human volunteer brain tissue subjected to an abrupt neck rotation, at different impact stages, row B) the detail of local strain distributions of the region indicated in row A, and row C) the experimental strain distribution computed by digital image correlation and plotted on the undeformed configuration of the magneto-active substrate.
Fig. 12. Shows a distribution of the strain magnitude and principal traction forces on the contact surface between the multifunctional substrate and the biological material when subjected to the action of two and four permanent magnets, respectively.
Fig. 13. Shows the polarization dynamics in the 3 regions showed in figure 12, where a polar distribution of cell orientation is shown before and after stimulation.
Fig. 14. Shows a scheme of one possible manufacturing process of the magneto-active substrate.

### PREFERRED EMBODIMENTS OF THE INVENTION

The invention relates to a magneto-mechanical stimulation system for non-invasive and real-time generation and control of complex strain patterns within biological materials (3).

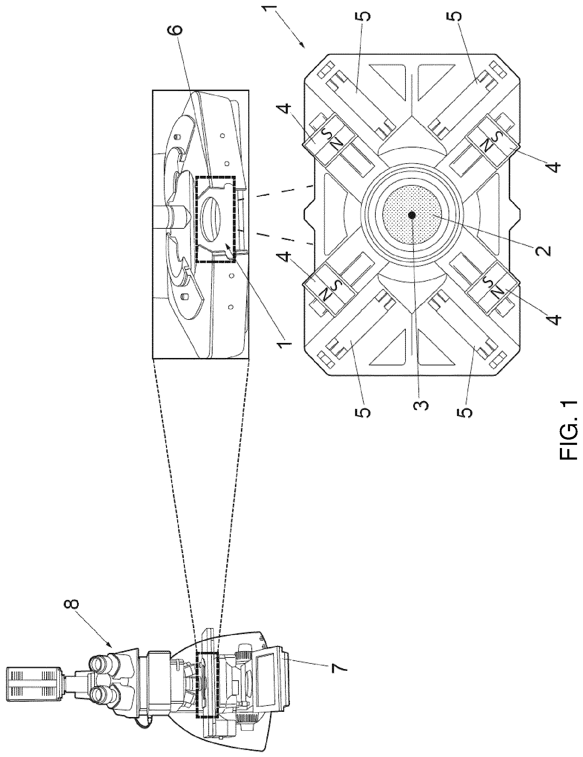

Figure 1 shows an exemplary embodiment of the stimulation system of the invention. The system of figure 1 comprises a non-ferromagnetic incubator (6) system designed to hold a magneto-responsive substrate (2). Biological material (3) is cultured on the substrate (2) so that the strain pattern generated on the substrate (2) is transferred to the cultured biological material (3). Preferably, a collagen coating is added on the surface of the substrate (2) wherein the biological material (3) is cultured.

The magneto-responsive substrate (2) comprises a polymeric matrix and a plurality of micron-size magnetic particles. More particularly, in this case, the polymeric matrix is a soft elastomer with a plurality of magnetic particles, i.e., a magnetorheological elastomer (MRE).

The system also comprises a magnetic stimulation device (1) which comprises in this case four magnets (4) placed on the incubator (6) around the magneto-responsive substrate (2) and configured to generate a magnetic field which acts over the magneto-responsive substrate (2). The magnetic stimulation device (1) also comprises four motors (5) connected each one to one of the magnets (4) to displace them.

Also, the system comprises an imaging module (8), which in this case is an upright fluorescence microscope (with ceramic objectives) for measuring the shape of the biological material (3) under the magnetic field generated by the magnetic stimulation device (1).

As a second alternative, a multi-compartment MRE can be used manufacturing its central region without magnetic particles. This central transparent region allows for phase contrast imaging on any upright or inverse microscope, making possible to record the cellular dynamics along the whole temporal event.

As a third alternative, a magneto-active hydrogel can be used combining a hydrogel matrix (or ferrogel) with micron- or nano-size magnetic particles embedded in it. This allows culturing biological material (3) within the active substrate (2), allowing for three-dimensional in-vitro tests.

The system further comprises a computing module (7) to determine the position of the magnets (4) for controlling the magnetic field generated. Said computing module (7) uses a 3D finite element module for performing computational simulations, thus, obtaining the permanent magnets' (4) relative positions to generate a given magnitude and direction of the magnetic field within the magneto-active substrate (2). The target magnetic field is estimated by multi-physical computational modelling to induce deformation patterns within the magneto-active substrate (2) to be transmitted to the biological system cultured.

As a second alternative for the computing module (7), artificial intelligence algorithms may be used for in situ design of experimental conditions, which can be changed along with the assay on demand. The algorithms take information from a database with experimental data providing local strain distributions as a function of the substrate (2) used and the permanent magnets' (4) positions.

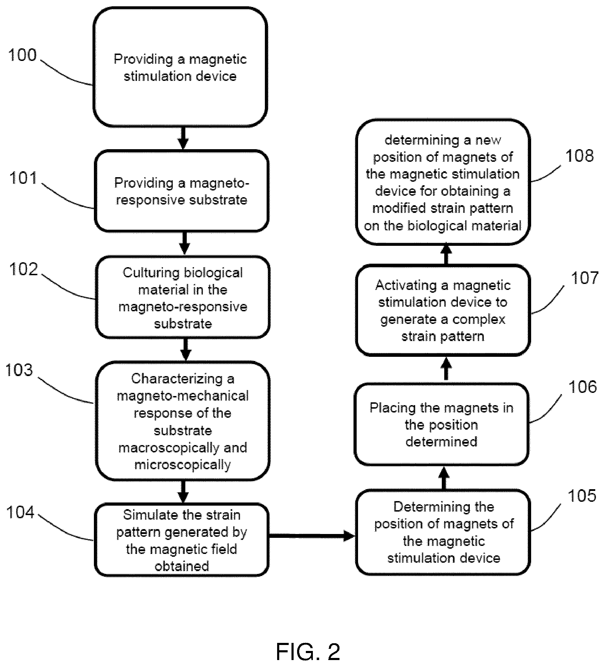

Figure 2 shows the steps of a method for generating and controlling complex strain patterns on biological materials (3). The method presented comprises a step of providing a magnetic stimulation device (1) and a magneto-responsive substrate (2). Then, biological material (3) is cultured on the magneto-responsive substrate (2).

The position of the magnets (4) of the magnetic stimulation device (1) is determined for obtaining a defined strain pattern on the biological material (3). In this step, simulations are carried out (or, alternatively, the artificial intelligent algorithms are used) for determining the magnets (4) position by determining the magnetic field generated as a function of the magnets' (4) position.

Also, the strain pattern generated on the substrate (2) by the magnetic field is simulated by introducing the magneto-mechanical response of the substrate (2) as an input of said simulations. For obtaining said magneto-mechanical response, a characterization of the substrate (2) is performed, both, macroscopically, by using a magneto-mechanical rheometer under uniaxial compression and shear deformation modes, and microscopically, by conducting nanoindentation tests at different rate conditions.

The method, then, comprises the steps of placing the magnets (4) in the position determined by means of the motors (5) of the magnetic stimulation device (1) and activating the magnetic stimulation device (1) to generate a complex strain pattern in the magneto-responsive substrate (2) and consequently in the biological material (3).

Also, the method can further comprise the steps of determining a new position of magnets (4) of the magnetic stimulation device (1) for obtaining a modified strain pattern on the biological material (3). In this way the strain pattern previously generated can be controlled on the fly by moving the magnets (4) of the magnetic stimulation device (1) to the new position determined.

In this example, the magneto-mechanical response of the substrate (2) is characterized accounting for twelve different combinations of the polymeric mixing ratio (i.e., matrix stiffness) and particles' volume fraction. A priori, softer polymeric matrices and higher particles' content lead to higher magnetorheological effects (i.e., mechanical deformation under magnetic fields and/or stiffness changes).

The magnetic problem must be solved macroscopically as it depends on the nature (i.e., permanent magnet, coil) and location of the magnetic sources as well as on the nature (i.e., material properties, geometry) and location of the substrate (2). The macroscopic response of the substrate (2) is thus governed by a strong structural component.

To evaluate such a macrostructural response, experiments in a magneto-mechanical rheometer are performed. These experiments allow for testing the substrates (2) under uniaxial compression and shear deformation modes in a wide range of magnetic field conditions.

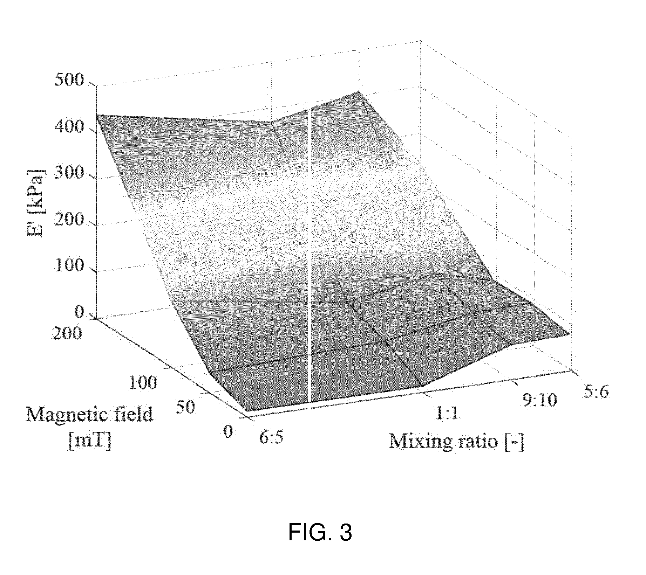

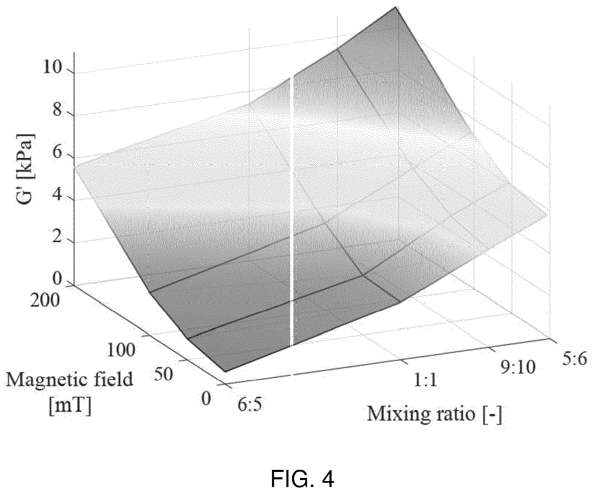

An example of the magneto-mechanical characterization at the macroscale, by using a rheometer at quasi-static conditions and for specific magneto-active substrates (2), is shown in Figures 3 and 4. Figure 3 shows the characterization under uniaxial compression loading, by representing the storage Young's modulus on the magnetic field applied for different magneto-active substrates (2) (using same polymeric matrix at different mixing ratios). Figure 4 shows the characterization under shear loading, representing the storage shear modulus depending on the magnetic field applied for different magneto-active substrates (2) (using same polymeric matrix at different mixing ratios).

Also, it must be noted that the biological material (3) cultured on the magneto-active substrate (2) consists of individuals with a characteristic length in the range of microns. Therefore, the stiffness, forces and deformations transmitted to the cells are related to local micron-size responses.

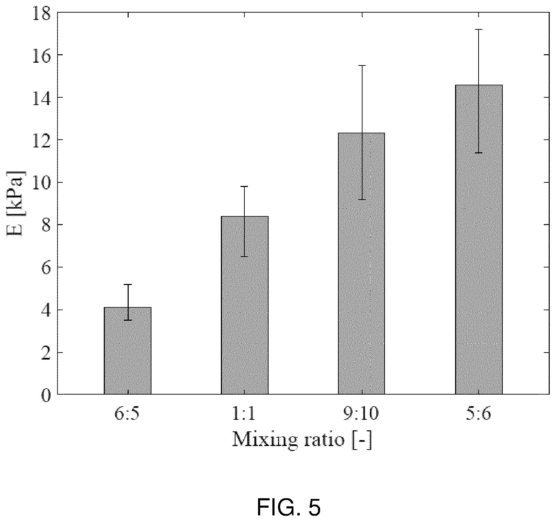

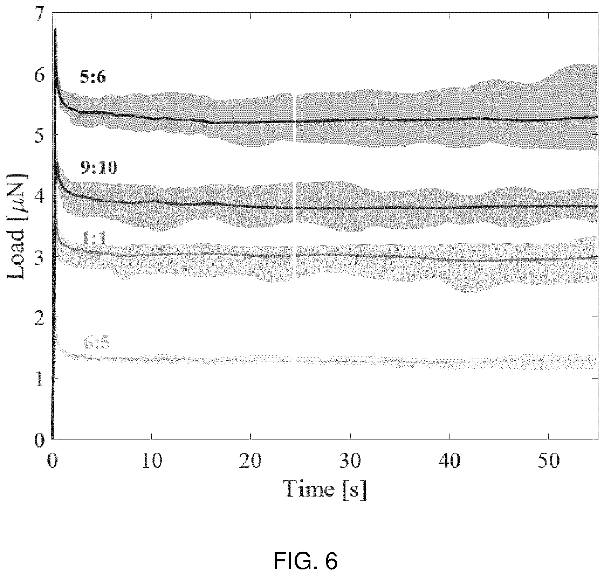

The microstructural response is evaluated by conducting nanoindentation tests at different rate conditions. The results are shown in Figures 5 and 6. The results of the quasi-static nanoindentation tests are shown in Figure 5, by representing the Young's modulus for different magneto-active substrates (2) (using same polymeric matrix at different mixing ratios).

Figure 6 shows the relaxation response depending on the mixing ratio used for the substrate (2), representing the load versus time relaxation curves.

Note that the microscopic measurements are taken on the polymeric phase and the values measured did not show significant changes when varying the particles' content or applying external magnetic fields.

In this regard, when subjected to an axial magnetic field, the magneto-active substrates (2) experienced a significant increase in stiffness that is stronger in compression than in shear loading. This effect is explained by the application of the magnetic field along the axial direction during compression loading, leading to the structural, macroscopic, response of the magneto-active substrate (2) to govern its behavior. Under shear loading the magnetic fields play a lower role on the substrate's (2) mechanical performance.

The macroscopic, i.e., rheological, experiments showed a magnetorheological effect (i.e., increase in stiffness) of up to 1.7 times for 50 mT and 9.3 times for 200 mT. Note that this stiffening refers to a macrostructural effect and does not imply microstructural stiffening of the polymeric matrix. The nanoindentation tests provided a local stiffness ranging from 4.1 to 14.6 kPa depending on the mixing ratio, and a characteristic relaxation time in the order of ~0.5-1.0 s.

Another important fact is that the collagen coating is found to play a negligible role in material stiffness at both macro and microstructural responses. This fact indicates that coating does not significantly affect the transmitted forces from the substrate (2) to the biological matter. The relaxation times of MREs with higher particles' content, at the macroscopic scale, were measured reporting a significant increase up to ~10 s.

From the results provided in Figures 3, 4, 5 and 6, it is inferred a promising magnetostrictive response of the active substrates (2) (i.e., mechanical deformation under an external magnetic field), but a slight increase in microstructural stiffness (i.e., stiffening of the polymeric phase) due to low strain-hardening (low nonlinear response).

For biomechanical stimulation, an evaluation at a microscale level (i.e., cell characteristic length) is needed. To assess the micro-magnetorheological effect, a nanoindentation system is coupled to the magnetic-stimulation device (1). This allows for measuring changes in stiffness due to the application of an external magnetic field.

The nanoindentation results within the central region showed negligible stiffness changes due to the external magnetic fields (as expected owing to the low strain-hardening of the polymeric matrix), as shown in Figures 5 and 6.

The step of using the characterization of the substrate (2) to simulate the strain pattern generated, could be performed imposing fixed boundary conditions, such that the substrate (2) presents a nonlinear mechanical behavior and suffers a variation in apparent material stiffness. Alternatively, said step could be performed imposing free boundary conditions, such that the substrate (2) mechanically deforms.

In this latter case, the mechanical deformation of the MRE due to the applied magnetic field can be evaluated.

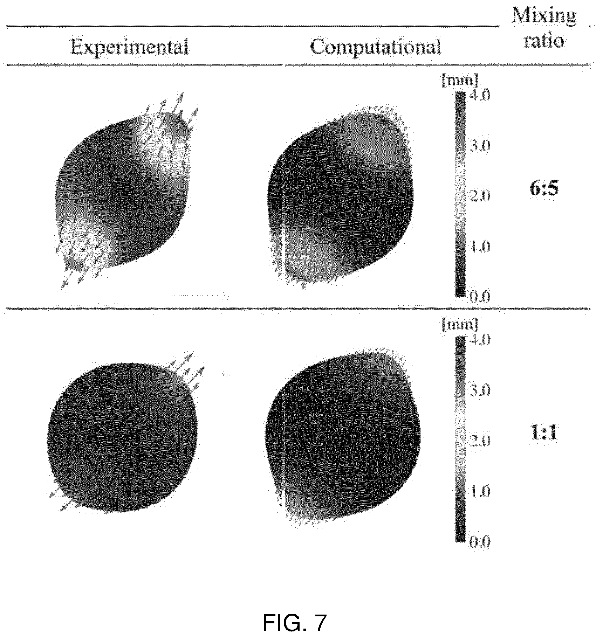

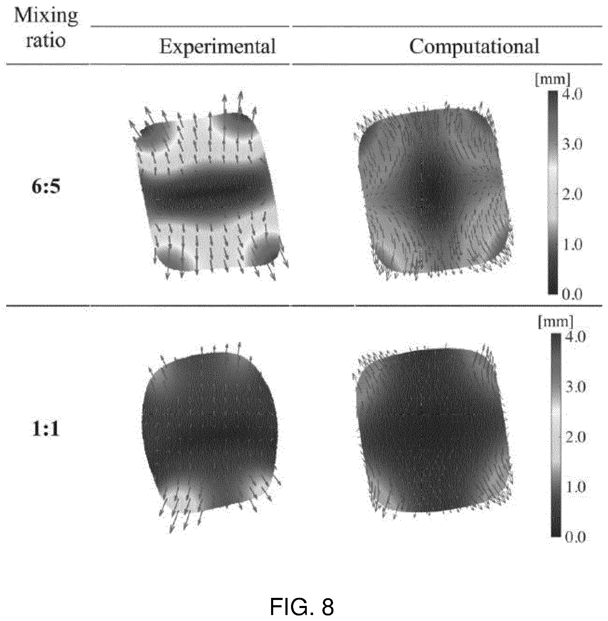

Figure 7 shows the effects of magnetic fields applied on MRE samples by means of magnetic stimulation. In particular, Figure 7 shows a comparison between experimental data and computational predictions by means of displacement fields, for an approach of two magnets (4) and for two different mixing ratios of the polymeric matrix (6:5 and 1:1). Figure 8 shows the same comparison for an approach of four magnets (4).

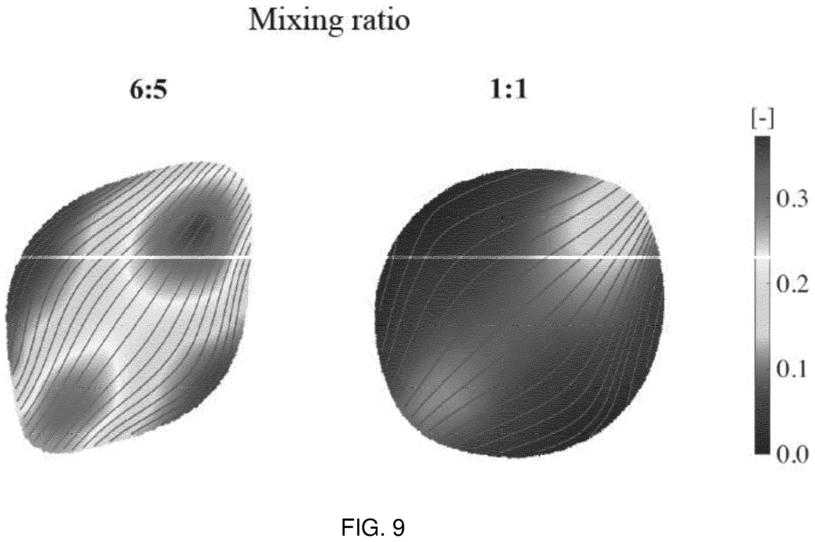

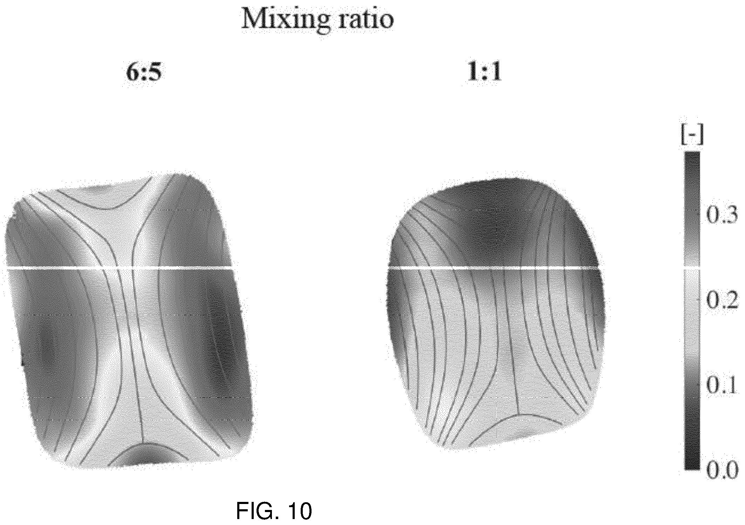

Figures 9 and 10 show experimental results obtained from digital image correlation of local mechanical deformation within MREs with different polymeric matrices and two different magnetic conditions (i.e., approach of two and four magnets (4)).

All these experiments showed significant mechanical deformations within the magneto-active substrate (2), as shown in Figures 9 and 10. In this case, Digital Image Correlation (DIC) was used to compute local deformation values. The plots shown in Figure 10 present the magnitude of the local deformations and principal strain lines to indicate the effective force direction that the cells sense.

In this regard, the magnetic-stimulation system can introduce deformation patterns in different directions of up to >30 %.

Microstructural and macrostructural experimental and computational data shows that the deformations develop as stretching along the main field direction. When the permanent magnets (4) approach the MRE, the magnetic particles start to magnetize leading to three main magneto-mechanical couplings:
i) dipole-dipole interaction between particles that result in attraction forces;
ii) paramagnetic torques that lead to the formation of chain-like particles distributions and their reorientation along the external magnetic field; and
iii) a strong attraction force between the particles and the magnet (4) itself.

The extremely soft nature of the polymeric matrix facilitates the rearrangement of particles favoring the latter mechanisms and, therefore, the resulting expansion of the substrate (2) towards the permanent magnet's location.

Table 1 shows the experimental results for an specific magneto-active substrate (2), thus, providing the stiffness and deformation ranges that can be reached with the magnetic stimulation system as a function of the manufacturing conditions of the substrate (2).

This table presents MRE samples' macroscopic and microscopic mechanical stiffnesses with different manufacturing parameters: the magnetic particles' volume fraction and polymeric mixing ratio. These mechanical properties are given for different magnetic conditions and are referred to as: i) macroscopic stiffness: shear modulus measured from magneto-rheological testing; ii) microscopic stiffness: Young's modulus obtained from nanoindentation with and without the presence of different sets of permanent magnets (4).

In addition, this table summarizes the local mechanical deformations within the MRE region occupied by cells when using the stimulation system.

**Table 1.**

| | **Multifunctional response of MRE samples** | | | | | |
|---|---|---|---|---|---|---|
| | **Magnetic conditions** | **Particles' volume fraction** | **Polymeric mixing ratio** | | | |
| | | | **6:5** | **1:1** | **9:10** | **5:6** |
| **Macroscopic stiffness (shear modulus) [kPa]** | 0 mT | 15 % | 0.4 | 1.5 | 2.9 | 3.2 |
| | | 30 % | 0.6 | 2.7 | 4.6 | 5.8 |
| | 50 mT | 15 % | 0.7 | 1.8 | 3.2 | 3.4 |
| | | 30 % | 1.0 | 2.8 | 5.0 | 6.2 |
| **Microscopic stiffness (Young's modulus) [kPa]** | Independent 0-50 mT | Independent 0-30 % | 4.1 | 8.4 | 12.3 | 14.6 |
| **Local deformation at MRE center [%]** | 2 magnets | 15 % | 14.3 | 4.0 | 1.7 | 1.2 |
| | | 30 % | 21.4 | 8.5 | 1.8 | 1.2 |
| | 4 magnets | 15 % | 16.4 | 3.4 | 1.4 | 1.1 |
| | | 30 % | 24.8 | 13.3 | 1.5 | 1.0 |

The system has been tested to reproduce brain strain patterns during closed head impact, one of the most complex mechanical scenarios as strain distributions evolve leading to complex non-symmetric strain distributions.

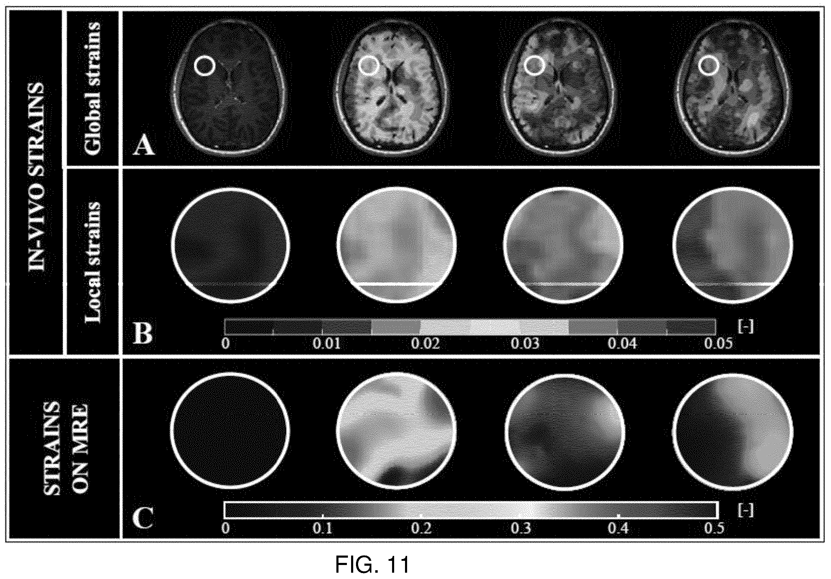

To this end, in vivo mechanical deformations of the brain tissue when subjected to a head impact scenario (neck abrupt rotation) has been obtained from literature (being limited to low strains to avoid volunteer injury). Figure 11 shows a proof of concept to evaluate the versatility of the system to simulate complex heterogeneous strain distributions.

The row A of Figure 11 shows an in vivo strain distribution within an axial view of a human volunteer brain tissue subjected to an abrupt neck rotation, at different impact (deformation) stages.

Row B of Figure 11 shows the detail of local strain distributions of the region indicated in row A with a circle.

Row C of Figure 11 shows the experimental strain distribution computed by digital image correlation and plotted on the undeformed configuration of the MRE substrates (2). It has to be noted that the experiments on the MREs represented in row C aim at reproducing the in vivo strain patterns but with an amplification of ten times their magnitude to reach strain values above injury thresholds in traumatic brain injury (≈ 21%).

Note that this adds complexity as it requires larger nonlinear deformation patterns. To do so, firstly, it is identified the relative positions of the magnets (4) that produce such targeted local heterogeneous strains fields, by running a complete set of computational simulations that provide strain measurements in local points of the MRE substrate (2), as explained before.

These local strains are compared with local measurements from the experimental data to find the solution that better reproduces the in vivo scenario. Then, the obtained magnets (4) positions are experimentally evaluated to validate the approach.

All in all, the magneto-mechanical system can reproduce heterogeneous strain distributions close to the real-brain ones, while allowing for amplifying their magnitude by ten times (thus approaching strain thresholds leading to injury). The experimental strains are represented on the undeformed configuration by means of Green-Lagrange strain, to provide a fair comparison with the in vivo experimental data.

A set of in vitro assays on cellular alignment of human dermal fibroblasts (hFBs) due to mechanical stimulation is included to demonstrate the ability of the system defined to transmit mechanical forces to biological material (3), i.e. cellular systems.

hFBs are particularly interesting as they play an essential role in the development and repair of tissues. Some state-of-the-art works are focused on analyzing the orientation of hFBs responding to mechanical stimulation (cyclic biaxial). These orientation or polarization processes are of high interest in mechanobiology.

Nevertheless, the published studies rarely alternate different mechanical deformation modes within the same assay and are usually limited to either uniaxial or biaxial loading. However, during pathophysiological conditions such as acute injuries, heterogeneous tissue stiffening is associated with changes in the ECM composition. These changes disrupt the mechanical homeostasis that underlies healthy tissue architecture and function, leading to alterations in the cell-generated forces and cellular mechanical properties.

Therefore, subsequent changes in the substrate (2) result in heterogeneous strain patterns and temporal-varying interaction forces that affect cellular organization and behavior. In this regard, the resultant cell polarization plays a relevant role in different processes such as collective migration, where a homogeneous orientation of the individuals facilitates mechanical propulsion forces and better intercellular communication.

The response of primary dermal hFBs, cultured on soft MREs (0.6 kPa shear stiffness), to heterogeneous strain distributions is evaluated. To this end, hFBs are cultured on the MRE substrates (2).

Then, the cellular system is subjected to different magneto-mechanical conditions, which correspond to the main cases studied, i.e., two and four sets of permanent magnets (4). Although heterogeneous strain patterns are reached in both cases, the two-magnets (4) scenario approaches to uniaxial loading and the four-magnets (4) scenario approaches to biaxial loading.

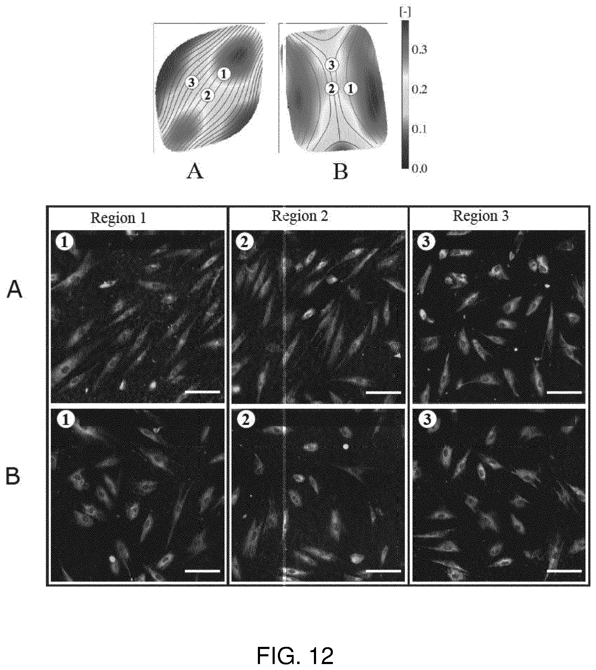

All these tests were analyzed associating cellular polarization with local mechanical conditions and evaluating cell viability. Local cell analyses were conducted on MRE regions, shown in Figure 12, with radial distances from the center below 7.5 mm, thus ensuring negligible strain deviations. Region 1 represents the highest local strain norm (26.0% and 28.9% for two magnets (4) and four magnets (4), respectively), region 2 the center of the MRE sample (21.4% and 24.8% for two magnets (4) and four magnets (4), respectively), and region 3 the lowest local strain norm among them (13.5% and 20.4% for two magnets (4) and four magnets (4), respectively).

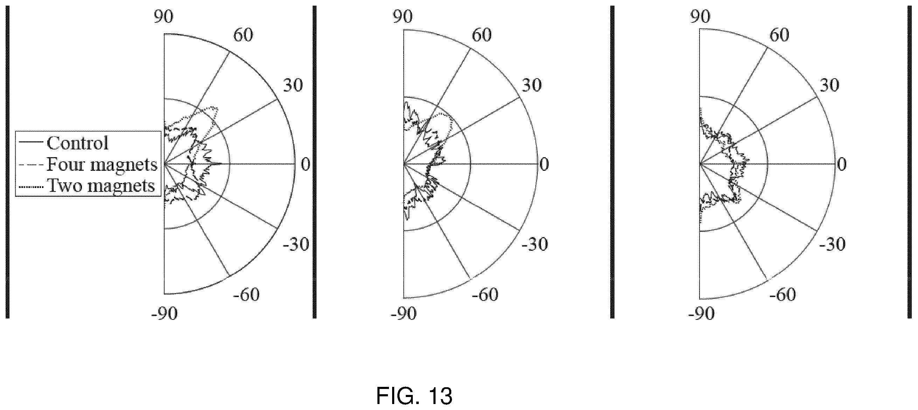

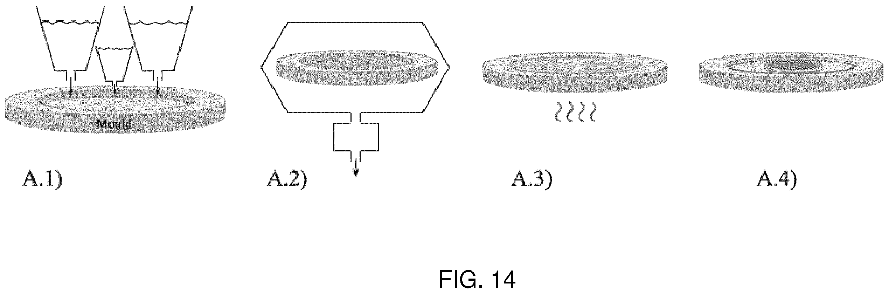

Figures 12 and 13 show a proof of concept to evaluate the ability of the system to transmit forces to the biological material (3), showing cellular polarization dynamics before and after magneto-mechanical stimulation.

Figure 12 shows a distribution of the strain magnitude and principal traction forces on the contact surface between the multifunctional substrate (2) and the biological material (3) when subjected to the action of two (A) and four (B) permanent magnets (4), respectively. Figure 12 also shows the regions which have been analyzed.

Figure 13 shows the polarization dynamics in the 3 regions showed in Figure 12, where a polar distribution of cell orientation is shown before and after stimulation. These results are for 2-hours stimulation. No significant differences between 2- and 24-hours stimulations are observed.

The results presented in Figure 13 indicate a sound transmission of the mechanical forces generated in the substrate (2) to the cells. In the two-magnets (4) stimulation, the effect of local strains is translated into cell alignment towards the principal stretching direction and modulated by the magnitude of these local deformations. Thus, region 1 presents higher cell polarization than region 2, and the latter presents higher polarization than region 3.

Moreover, the four-magnets (4) stimulation does not present cell alignment in a clear preferred orientation. All these features are shown by the microscopic images and orientation distributions histograms shown in Figures 12 and 13.

In Figure 12, it has to be noted that the magnetic particles present certain autofluorescence that in some figures overlaps with the cellular cytoskeleton stained in green.

These features suggest that the cellular alignment is determined by two main variables: the deformation state defined as the relation between principal strain components; and the magnitude of such strain components. Thus, a strain state close to uniaxial tension (i.e., one main principal component) promotes cell alignment, and this is modulated by the strain magnitude. However, if the cellular system is exposed to deformation states with no clear principal direction (i.e., close to biaxial loading), the cells do not polarize in a preferred direction.

The capability of the system to transmit dynamic loading to the biological material (3) is demonstrated. Therefore, the system is proved to be able to reproduce strain patterns on a soft substrate (2) but also to interact with the biological system cultured on it, allowing for studying the effects of heterogeneous mechanical scenarios on biological processes.

The viability and versatility of the complete experimental-computational framework for in vitro mechanical stimulation of cellular systems have been assessed by the two applications described. Firstly, the ability of the system to reproduce complex mechanical scenarios has been demonstrated by simulating a set of local strain patterns occurring within the brain tissue during a head impact. And the capability of the framework to transmit mechanical forces to cellular systems has been evaluated by subjecting primary human dermal fibroblasts (hFBs) to magneto-mechanical loading.

Figure 14 shows a manufacturing method of magneto-responsive MRE substrates (2). Therefore, the manufacturing method of the MRE substrate (2) comprises the steps of mixing polymeric material and magnetic particles. Then, a degassing process is carried out by means of a vacuum chamber applying vacuum by using a pump for 20 minutes. After that, a curing step is performed heating to 80ºC for 2 hours, and then, a die-cut is done and a functionalization applying a collagen coating.

## Claims

1. Method for generating and controlling temporally evolving mechanical stiffness and/or complex strain patterns on biological materials (3) comprising the steps of:
- providing a magnetic stimulation device (1);
- providing a magneto-responsive substrate (2);
- culturing biological material (3) in the magneto-responsive substrate (2);
- determining the position of magnets (4) of the magnetic stimulation device (1) for obtaining a defined strain pattern on the biological material (3), by simulating the magnetic field obtained as a function of the magnets' (4) position, by using finite element simulations or machine learning algorithms fed by a comprehensive experimental characterization of several magneto-active samples;
- placing the magnets (4) in the position determined by means of at least one motor (5) of the magnetic stimulation device (1);
- activating a magnetic stimulation device (1) to generate a complex strain pattern in the magneto-responsive substrate (2) and consequently in the biological material (3).

2. Method according to claim 1, further comprising the steps of characterizing a magneto-mechanical response of the substrate (2) macroscopically, by using a magneto-mechanical rheometer under uniaxial compression and shear deformation modes, and microscopically, by conducting nanoindentation tests at different rate conditions and using said characterization to simulate the strain pattern generated by the magnetic field obtained.

3. Method according to claim 2, wherein in the step of using said characterization to simulate the strain pattern generated:
- fixed boundary conditions are imposed, such that the substrate (2) presents a nonlinear mechanical behavior and suffers a variation in apparent material stiffness; or
- free boundary conditions are imposed, such that the substrate (2) mechanically deforms.

4. Method according to claim 2, further comprising the steps of determining a new position of magnets (4) of the magnetic stimulation device (1) for obtaining a modified strain pattern on the biological material (3), once the strain pattern generated is obtained for controlling said strain pattern on the fly and moving the magnets (4) of the magnetic stimulation device (1) to the new position determined.

5. Magneto-mechanical stimulation system for generating complex strain patterns in biological materials (3) comprising:
- a magneto-responsive substrate (2) comprising a polymeric matrix and a plurality of micron-size magnetic particles configured to hold biological material (3);
- a holder (6) for placing the magneto-responsive substrate (2);
- a magnetic stimulation device (1) comprising at least one motor (5) and at least two magnets (4) placed on the holder (6) around the magneto-responsive substrate (2) and configured to generate a complex strain pattern on the biological material (3) by generating a magnetic field which acts over the magneto-responsive substrate (2), wherein the at least one motor (5) is connected to the magnets (4) to displace them;
- a computing module (7) to determine the position of the magnets (4) for controlling the magnetic field generated;
- an imaging module (8) for measuring the shape of the biological material (3) under the magnetic field generated; and
- an interface module for imposing magneto-mechanical dynamic conditions on the computing module (7) by using the measurements obtained from the imaging module (8) and comprising a 3D finite element module or artificial intelligence algorithms for controlling and predicting the deformation patterns transmitted to the cells during the application of the magnetic field.

6. Stimulation system, according to claim 5, wherein the magneto-responsive substrate (2) is a magnetorheological elastomer (MRE).

7. Stimulation system according to claim 5, wherein the polymeric matrix is made of a magneto-active hydrogel or Dowsil CY52-276 (PDMS) and is filled with micron-size magnetic particles of carbonyl iron powder.

8. Stimulation system according to claim 5, wherein the surface in contact with the cultured cellular system is covered with a collagen coating.

9. Stimulation system according to claim 5, wherein the magnetic stimulation device (1) comprises four independently controllable sets of permanent magnets (4) surrounding the substrate (2), with two sets aligned along an axis, forming two axes which lie orthogonal to control the field in each direction, and a rotation mechanism to allow an azimuthal rotation.

10. Stimulation system according to claim 5, further comprising a magneto-mechanical rheometer and/or a nanoindentation system to characterize the substrate macroscopically and/or microscopically (2).

11. Stimulation system according to claim 5, wherein the holder (6) and the imaging module (8) are comprised of an incubator (6).

12. A computer program adapted to perform the steps of the method of any of claims 1 to 4 by using the computing module (7) of the stimulation system of any of claims 5 to 11.

13. A computer-readable storage medium comprising the computer program of claim 12.

## Patentansprüche

1. Verfahren zur Erzeugung und Steuerung von sich zeitlich entwickelnden mechanischer Steifigkeit und/oder komplexen Dehnungsmustern auf biologischen Materialien (3), das die folgenden Schritte umfasst:
- Bereitstellen einer magnetischen Stimulationsvorrichtung (1);
- Bereitstellen eines auf Magnetismus reagierenden Substrats (2);
- Kultivieren von biologischem Material (3) in dem auf Magnetismus reagierenden Substrat (2);
- Bestimmen der Position von Magneten (4) der magnetischen Stimulationsvorrichtung (1), um ein definiertes Dehnungsmuster auf dem biologischen Material (3) zu erhalten, indem das in Abhängigkeit von der Position der Magneten (4) erhaltene Magnetfeld unter Verwendung von Finite-Elemente-Simulationen oder Maschinenlernalgorithmen simuliert wird, die durch eine umfassende experimentelle Charakterisierung mehrerer magnetoaktiver Proben gespeist werden;
- Platzieren der Magnete (4) in der durch mindestens einen Motor (5) der magnetischen Stimulationsvorrichtung (1) bestimmten Position;
- Aktivieren einer magnetischen Stimulationsvorrichtung (1), um ein komplexes Dehnungsmuster in dem auf Magnetismus reagierenden Substrat (2) und folglich in dem biologischen Material (3) zu erzeugen.

2. Verfahren nach Anspruch 1, ferner umfassend die Schritte des Charakterisierens, auf makroskopischer Ebene, einer magnetomechanischen Reaktion des Substrats (2) unter Verwendung eines magnetomechanischen Rheometers in den Betriebsarten uniaxiale Kompression und Scherverformung und auf mikroskopischer Ebene durch Durchführen von Nanoindentationstests bei verschiedenen Geschwindigkeitsbedingungen und unter Verwendung der Charakterisierung, um das durch das erhaltene Magnetfeld erzeugte Dehnungsmuster zu simulieren.

3. Verfahren nach Anspruch 2, wobei in dem Schritt des Verwendens der Charakterisierung, um das generierte Dehnungsmuster zu simulieren:
- festgelegte Randbedingungen auferlegt werden, so dass das Substrat (2) ein nichtlineares mechanisches Verhalten aufweist und eine Variation der scheinbaren Materialsteifigkeit erfährt; oder
- freie Randbedingungen auferlegt werden, so dass sich das Substrat (2) mechanisch verformt.

4. Verfahren nach Anspruch 2, ferner umfassend die Schritte des Bestimmens einer neuen Position der Magnete (4) der magnetischen Stimulationsvorrichtung (1), um ein modifiziertes Dehnungsmuster auf dem biologischen Material (3) zu erhalten, sobald das erzeugte Dehnungsmuster erhalten wird, um das Dehnungsmuster im laufenden Betrieb zu steuern und die Magnete (4) der magnetischen Stimulationsvorrichtung (1) in die neu bestimmte Position zu bewegen.

5. Magnetomechanisches Stimulationssystem zur Erzeugung komplexer Dehnungsmuster in biologischen Materialien (3), umfassend:
- ein auf Magnetismus reagierendes Substrat (2), das eine polymere Matrix und eine Vielzahl von mikrometergroßen magnetischen Partikeln umfasst, die dazu konfiguriert sind, biologisches Material (3) aufzunehmen;
- einen Halter (6) zum Platzieren des auf Magnetismus reagierenden Substrats (2);
- eine magnetische Stimulationsvorrichtung (1), die mindestens einen Motor (5) und mindestens zwei Magnete (4) umfasst, die auf dem Halter (6) um das auf Magnetismus reagierende Substrat (2) herum platziert und dazu konfiguriert sind, ein komplexes Dehnungsmuster auf dem biologischen Material (3) zu erzeugen, indem ein Magnetfeld erzeugt wird, das auf das auf Magnetismus reagierende Substrat (2) wirkt, wobei der mindestens eine Motor (5) mit den Magneten (4) verbunden ist, um sie zu verschieben;
- ein Rechenmodul (7) zum Bestimmen der Position der Magneten (4) zum Steuern des erzeugten Magnetfeldes;
- ein Bildgebungsmodul (8) zum Messen der Form des biologischen Materials (3) unter dem erzeugten Magnetfeld; und
- ein Schnittstellenmodul, um dem Rechenmodul (7) unter Verwendung der vom Bildgebungsmodul (8) erhaltenen Messungen magnetomechanische dynamische Bedingungen aufzuerlegen, und das ein 3D-Finite-Elemente-Modul oder Algorithmen der künstlichen Intelligenz umfasst, um die während der Anwendung des Magnetfeldes auf die Zellen übertragenen Verformungsmuster zu steuern und vorherzusagen.

6. Stimulationssystem nach Anspruch 5, wobei das auf Magnetismus reagierende Substrat (2) ein magnetorheologisches Elastomer (MRE) ist.

7. Stimulationssystem nach Anspruch 5, wobei die polymere Matrix aus einem magnetoaktiven Hydrogel oder Dowsil CY52-276 (PDMS) hergestellt ist und mit mikrometergroßen magnetischen Partikeln aus Carbonyleisenpulver gefüllt ist.

8. Stimulationssystem nach Anspruch 5, wobei die mit dem kultivierten zellulären System in Kontakt stehende Oberfläche mit einer Kollagenbeschichtung bedeckt ist.

9. Stimulationssystem nach Anspruch 5, wobei die magnetische Stimulationsvorrichtung (1) vier unabhängig voneinander steuerbare Sätze von Permanentmagneten (4) umfasst, die das Substrat (2) umgeben, wobei zwei Sätze entlang einer Achse ausgerichtet sind und zwei Achsen bilden, die orthogonal zueinander liegen, um das Feld in jeder Richtung zu steuern, sowie einen Drehmechanismus, um eine azimutale Drehung zu ermöglichen.

10. Stimulationssystem nach Anspruch 5, ferner umfassend ein magnetomechanisches Rheometer und/oder ein Nanoindentationssystem, um das Substrat (2) makroskopisch und/oder mikroskopisch zu charakterisieren.

11. Stimulationssystem nach Anspruch 5, wobei der Halter (6) und das Bildgebungsmodul (8) einen Inkubator (6) umfassen.

12. Computerprogramm, das dazu angepasst ist, die Schritte des Verfahrens nach einem der Ansprüche 1 bis 4 unter Verwendung des Rechenmoduls (7) des Stimulationssystems nach einem der Ansprüche 5 bis 11 durchzuführen.

13. Computerlesbares Speichermedium, das das Computerprogramm nach Anspruch 12 umfasst.

## Revendications

1. Procédé de génération et de contrôle de la rigidité mécanique évoluant dans le temps et/ou de modèles de contrainte complexes sur des matériaux biologiques (3) comprenant les étapes consistant à :
- fournir un dispositif de stimulation magnétique (1) ;
- fournir un substrat magnéto-réceptif (2) ;
- cultiver du matériau biologique (3) dans le substrat magnéto-réactif (2) ;
- déterminer la position des aimants (4) du dispositif de stimulation magnétique (1) pour l'obtention d'un modèle de contrainte défini sur le matériau biologique (3), en simulant le champ magnétique obtenu en fonction de la position des aimants (4), en utilisant des simulations par éléments finis ou d'algorithmes d'apprentissage automatique alimentés par une caractérisation expérimentale complète de plusieurs échantillons magnétoactifs ;
- placer les aimants (4) dans la position déterminée au moyen d'au moins un moteur (5) du dispositif de stimulation magnétique (1) ;
- activer un dispositif de stimulation magnétique (1) pour générer un modèle de contrainte complexe dans le substrat magnéto-réactif (2) et, par conséquent, dans le matériau biologique (3).

2. Procédé selon la revendication 1, comprenant en outre les étapes consistant à caractériser une réponse magnéto-mécanique du substrat (2) microscopiquement, en utilisant un rhéomètre magnéto-mécanique en mode de compression uniaxiale et de déformation par cisaillement, et au microscopiquement, en menant des essais de nanoindentation à différentes conditions de vitesse et en utilisant ladite caractérisation pour simuler le modèle de contrainte généré par le champ magnétique obtenu.

3. Procédé selon la revendication 2, dans lequel dans l'étape consistant à utiliser ladite caractérisation pour simuler le modèle de contrainte généré :
- des conditions aux limites fixes sont imposées, de telle sorte que le substrat (2) présente un comportement mécanique non linéaire et subit une variation de la rigidité apparente de matériau ; ou
- des conditions aux limites libres sont imposées, de telle sorte que le substrat (2) se déforme mécaniquement.

4. Procédé selon la revendication 2, comprenant en outre les étapes consistant à déterminer une nouvelle position d'aimants (4) du dispositif de stimulation magnétique (1) pour l'obtention d'un modèle de contrainte modifié sur le matériau biologique (3), une fois que le modèle de contrainte généré est obtenu pour le contrôle dudit de modèle de contrainte à la volée et le mouvement des aimants (4) du dispositif de stimulation magnétique (1) vers la nouvelle position déterminée.

5. Système de stimulation magnéto-mécanique pour la génération de modèles de contrainte complexes dans des matériaux biologiques (3) comprenant :
- un substrat magnéto-réactif (2) comprenant une matrice polymère et une pluralité de particules magnétiques de taille micrométrique configurées pour contenir du matériau biologique (3) ;
- un support (6) pour le placement du substrat magnéto-réactif (2) ;
- un dispositif de stimulation magnétique (1) comprenant au moins un moteur (5) et au moins deux aimants (4) placés sur le support (6) autour du substrat magnéto-réactif (2) et configurés pour générer un modèle de contrainte complexe sur le matériau biologique (3) en générant un champ magnétique qui agit sur le substrat magnéto-réactif (2), dans lequel au moins un moteur (5) est connecté aux aimants (4) pour les déplacer ;
- un module de calcul (7) pour déterminer la position des aimants (4) pour le contrôle du champ magnétique généré ;
- un module d'imagerie (8) pour la mesure de la forme du matériau biologique (3) sous le champ magnétique généré ; et
- un module d'interface pour l'imposition de conditions dynamiques magnéto-mécaniques sur le module de calcul (7) en utilisant des mesures obtenues du module d'imagerie (8) et comprenant un module d'éléments finis 3D ou des algorithmes d'intelligence artificielle pour le contrôle et la prédiction des modèles de déformation transmis aux cellules pendant l'application du champ magnétique.

6. Système de stimulation, selon la revendication 5, dans lequel le substrat magnéto-réactif (2) est un élastomère magnétorhéologique (EMR).

7. Système de stimulation selon la revendication 5, dans lequel la matrice polymère est constituée d'un hydrogel magnéto-actif ou de Dowsil CY52-276 (PDMS) et est remplie de particules magnétiques de taille micrométrique de poudre de fer carbonyle.

8. Système de stimulation selon la revendication 5, dans lequel la surface en contact avec le système cellulaire cultivé est recouverte d'une couche de collagène.

9. Système de stimulation selon la revendication 5, dans lequel le dispositif de stimulation magnétique (1) comprend quatre ensembles d'aimants permanents (4) contrôlables indépendamment entourant le substrat (2), deux ensembles étant alignés le long d'un axe, formant deux axes qui sont situés orthogonaux pour contrôler le champ dans chaque direction, et un mécanisme de rotation pour permettre une rotation azimutale.

10. Système de stimulation selon la revendication 5, comprenant en outre un rhéomètre magnéto-mécanique et/ou un système de nanoindentation pour caractériser le substrat macroscopiquement et/ou microscopiquement (2).

11. Système de stimulation selon la revendication 5, dans lequel le support (6) et le module d'imagerie (8) sont constitués d'un incubateur (6).

12. Programme d'ordinateur adapté pour réaliser les étapes du procédé selon l'une quelconque des revendications 1 à 4 en utilisant le module de calcul (7) du système de stimulation selon l'une quelconque des revendications 5 à 11.

13. Support de stockage lisible par ordinateur comprenant le programme informatique selon la revendication 12.
